# EUROPEAN PATENT SPECIFICATION

(11) **EP 3 685 797 B1**
(45) Date of publication and mention of the grant of the patent: **29.06.2022**
(21) Application number: 18859076.4
(22) Date of filing: 23.05.2018
(51) Int. Cl.: A61C 13/00, G06T 17/00, A61C 11/00, A61C 9/00, A61C 7/00

(54) **METHOD FOR USING A DYNAMIC VIRTUAL ARTICULATOR FOR SIMULATING OCCLUSION WHEN DESIGNING A DENTAL PROSTHESIS FOR A PATIENT, AND DATA CARRIER**
VERFAHREN ZUR VERWENDUNG EINES DYNAMISCHEN VIRTUELLEN ARTIKULATORS ZUR SIMULATION DER OKKLUSION BEIM ENTWURF EINER ZAHNPROTHESE FÜR EINEN PATIENTEN SOWIE DATENTRÄGER
PROCÉDÉ D'UTILISATION D'UN ARTICULATEUR VIRTUEL DYNAMIQUE POUR LA MODÉLISATION PAR IMITATION D'UNE OCCLUSION LORS DE LA CONCEPTION DE PROTHÈSE STOMATOLOGIQUES POUR UN PATIENT ET SUPPORT D'INFORMATIONS

(30) Priority: 20.09.2017 RU 2017132793
(43) Date of publication of application: 29.07.2020
(73) Proprietor: Obschestvo S Ogranichennoi Otvetstvennostyu "Avantis3d", Moscow 121205 (RU)
(72) Inventor: RYAKHOVSKY, Alexandr Nikolaevich, Moscow 117418 (RU); RYAKHOVSKY, Stanislav Aleksandrovich, Moscow 117418 (RU); VYKHODTSEVA, Maria Alexandrovna, Moscow 117418 (RU)
(74) Representative: Chimini, Francesco
(86) International application number: PCT/RU2018/050054
(87) International publication number: WO 2019/059813

(56) References cited:
- WO-A1-2009/133131
- WO-A1-2012/006696
- WO-A1-2013/123062
- RU-C1- 2 401 083
- RU-C2- 2 567 604
- RU-C2- 2 581 029
- US-A1- 2011 276 159
- US-A1- 2013 275 107

## Description

### FIELD OF THE INVENTION

The group of inventions relates to medical devices, namely, to the means for dynamic virtual articulation.

### BACKGROUND

Articulator, as a mechanical device, represents a simplified model of masticatory apparatus to the frames of which the dental models are fixed, and their movement is set by guides in the area of articular joints and by inclined faces of the incisal table. The inclination of the guides and faces is set based on anatomic-mean or individual values registered by means of axiography. Dental lab technician studies availability and sequence of contacts occurred on teeth and also on artificial teeth of the future denture structure by simulation of side and forward-backward movements from the position of the centric (habitual) occlusion.

The prior art discloses systems and methods for analyzing dynamic occlusion during replicated excursion of virtual three-dimensional representations of a dental patient's occlusal complex (US2013275107A1).

The prior art discloses the method of using the dynamic virtual articulator for occlusion simulation modeling at automated designing the artificial dentures and at planning the orthodontic treatment of patient's teeth, implemented by means of a computer (RU 2567604, 10.11), including: providing of the virtual articulator comprising the virtual 3D model of upper jaw and virtual 3D model of lower jaw, replicating the patient's upper and lower jaws respectively; providing the movement of the virtual upper jaw and virtual lower jaw relative to each other for simulation modeling of dynamic occlusion when contacts between teeth of the virtual upper jaw and virtual lower jaw occur, and this method includes prevention of intercrossing between virtual surface of the virtual upper jaw teeth and virtual surface of the virtual lower jaw teeth in case of the above mentioned contacts. Therefore, the virtual teeth act as physical impenetrable objects.

The defective features of the method known are as follows:
- the presented virtual articulator is in fact a digital copy of the mechanical articulator, which frame movements differ, to a certain extent, from actual movements of the lower jaw. The cause of such differences is that movement simulation is based on the movement of the mandibular axis and rotation of the objects around this axis. Provided that, the mandibular axis in the articulator is located in the area of the articulator's frames junction, however, there is no precisely fixed position of the mandibular axis for the dentofacial system; the lower jaw rotation axis changes its position in space and could be beyond the temporo-mandibular joint. The distance between articular joints in the known articulators is precisely fixed and corresponds to anatomic-mean values, while the nature of the lower jaw actual movements is determined by the distance between the mandible heads, which is specific to each patient;
- the known embodiments of the virtual articulator do not take into account the movements of the lower jaw articular heads and their relations with other elements of the temporo-mandibular joints, it is not possible to set the controllable clearance between the mandible heads and mandibular fossa, that takes place at actual movements of the lower jaw;
- the known embodiments of the virtual articulator provide simulation of relative movements of upper and lower jaws subject to mutual contact of teeth and it is not possible to set the controllable clearance between opposing teeth. It means the necessity to carry out iterative corrections when modeling teeth form or position in cases, when, for example, it is required to open the bite;
- the known embodiments of the virtual articulator do not provide a possibility of mutually causal planning of different dental treatments. Planning of different dental treatments is carried out in sequence: planning of each new treatment stage is based on the conditions achieved at the previous stage. Such traditional approach results in overlong total periods of combination dental treatment.

### SUMMARY OF THE INVENTION

Technical result is obtaining the maximum approximation of the virtual articulator, simulating actual processes taking place in the patient's mouth, that is achieved due to the following:
- virtual articulator enables to make movements replicating and simulating actual processes, taking place in the patient's mouth, since the individual distance between the mandible heads is set in contrast with the strictly set distance between the articular joints of the virtual articulator as a copy of the mechanical system in the known prior arts;
- virtual articulator enables to make movements replicating and simulating actual processes, taking place in the patient's mouth, since the movement simulation is not based on preliminary determination of mandibular axis and its subsequent movement with lower jaw virtual objects rotation around it, as is done in the known prior arts; contrary to this the movements of the virtual lower jaw are determined, on the one hand, at least by one of the contacts between virtual dental arches, on the other hand, by the set position of the patient's virtual mandible heads and controllable separation from virtual joints and virtual surfaces of its temporal bones (articular eminences and mandibular fossae). Therefore, relative movement of jaws is more realistic physiologically;

- virtual articulator enables to make movements replicating and simulating actual processes, taking place in the patient's mouth, since the movements of the virtual lower jaw are determined, on the one hand, at least by one of the contacts between virtual dental arches with penetration of the virtual models into each other or without it, as is done in the known prior arts, on the other hand, they could be determined by functional occlusion terminal positions (protrusion, laterotrusion, etc.), virtually set by an operator, at which the separation between dental arches (lack of contacts) is set in advance. Therefore, virtual correction of teeth form or position is carried out not iteratively with a lot of repetitions, as is done in the known prior arts, but straight away taking into account the set movements of the virtual lower jaw;
- virtual articulator is the virtual model not of physical mechanical system but of real masticatory apparatus of the patient.

Besides, the inventive method enables to reduce time expenditures for registration and virtual simulation of the lower jaw articulation due to lack of necessity to use special devices for registration of the lower jaw movements, an also due to decreasing of discomfort at registration of movements and additional errors caused by necessity to attach additional devices to teeth, as is done in the known prior arts.

The summary of the group of inventions developed by us is as follows.

Method of using the dynamic virtual articulator for occlusion modeling at changing the initial position of the lower jaw, its fragments, teeth position relative to each other, position and form of artificial teeth, implemented by means of a computer, includes the following stages: providing of the virtual articulator comprising the virtual 3D model of upper jaw and dental arch and virtual 3D model of lower jaw and dental arch, replicating the patient's upper jaw and teeth and lower jaw and teeth respectively, and providing the simulation movements of the virtual upper jaw and virtual lower jaw relative to each other for simulation modeling of dynamic occlusion at which penetrable and impenetrable contacts between teeth of the virtual upper jaw and virtual lower jaw could occur. Provided that, providing the virtual articulator includes virtual 3D models of the temporo-mandibular joints, replicating the mandible heads, mandibular fossae and articular eminences of the temporal bones, and simulation movements of the jaws relative to each other is preceded by clinical or virtual setting of initial and terminal positions. Besides, virtual surfaces of the mandible heads and virtual surfaces of the articular eminences and mandibular fossae of the patient's temporal bones are separated.

In particular embodiments of the method:
- virtual articulator additionally includes virtual models of 3D and 2D face images and 2D images of lateral and frontal teleroentgenograms;
- virtual models of teeth, lower jaw bone, teeth roots, soft tissues are painted in the color of real objects;
- virtual models of teeth, lower jaw, elements of the temporo-mandibular joint are obtained on the basis of direct or indirect scanning;
- scanning includes at least one image of optical scanning, ultrasonic scanning, X-ray tomography or magnetic resonance imaging;
- indirect scanning includes using of the dental arch impression;
- providing the simulation movements of the virtual lower jaw based on registration of different terminal positions of dental arches by means of intraoral scanning; and/or obtaining bite registers at specified different terminal positions, their subsequent scanning and sequential comparison of virtual objects of the lower dental arch, lower jaw, mandible heads;
- providing the simulation movements of the virtual lower jaw based on registration of its movements;
- registration of movements of the lower jaw, lower dental arch or its fragment, one or more reference points, fixed on the lower jaw or lower teeth, is effected by means of electromagnetic waves or ultrasonic waves;
- providing the simulation movements of the virtual lower jaw based on anatomic-mean parameters of articular and incisal guidance, lateral displacement;
- providing the simulation movements of the virtual lower jaw from the centric relation position;
- virtual position of centric relation is set clinically by direct or indirect registration;
- direct registration includes intraoral scanning, and indirect registration includes obtaining the bite registers, their scanning and subsequent comparison of virtual registers and upper jaw teeth, and then comparison of lower jaw virtual objects, related to the lower dental arch, and lower teeth impressions on virtual registers;
- virtual position of centric relation is set by operator manually or automatically based on anatomic-mean width of articular cavities, by virtual correction of the position of the mandible heads' virtual models and related lower jaw virtual objects, and by virtual correction of inter-incisal height or vertical relations of any other selected virtual opposing teeth;
- virtual position of centric relation is set by operator manually or automatically based on correction of the distance between masticatory muscles attachment points, by virtual correction of the lower jaw position;
- virtual position of centric relation is set clinically based on direct or indirect registration of the lower jaw position, at which the maximum masticatory force develops;
- direct registration includes intraoral scanning, and indirect registration includes obtaining the bite registers, their scanning and subsequent comparison of virtual registers and upper jaw teeth, and then comparison of lower jaw virtual objects, related to the lower dental arch, and lower teeth impressions on virtual registers;
- providing the simulation movements of the virtual lower jaw from the position of centric or habitual occlusion, centric relation, into the virtual terminal positions preliminarily set by operator, provided that, changing of the patient's teeth surface at virtual planning of their direct restoration, at virtual planning of their displacement at orthodontic treatment, at virtual planning of their indirect restoration at dental prosthetics using virtual models of artificial teeth, is carried out immediately taking into account the simulation movements of the virtual lower jaw with no need for additional iterations;
- providing the simulation movements of the virtual lower jaw from the position of centric or habitual occlusion, centric relation, when the occlusion contact points are set by changing the patient's teeth surface at virtual planning of their direct restoration, at virtual planning of their displacement at orthodontic treatment, at virtual planning of their indirect restoration at dental prosthetics using virtual models of artificial teeth;
- providing impenetrability of the patient's virtual teeth or virtual artificial teeth;
- providing penetrability of the patient's virtual teeth or virtual artificial teeth;
- virtual planning of teeth position orthodontic correction or teeth form prosthetic correction from the position of centric or habitual occlusion taking into account the simulation movements of the virtual lower jaw;
- virtual planning of teeth position orthodontic correction or teeth form prosthetic correction from the virtually set centric relation, taking into account the simulation movements of the virtual lower jaw;
- the method can include mutually causal virtual planning of teeth position orthodontic correction or teeth form and size prosthetic correction from the position of centric or habitual occlusion, from the virtually set centric relation, taking into account the final teeth position at orthodontic correction and taking into account the simulation movements of the virtual lower jaw;
- including mutually causal virtual planning of implantation taking into account the jaw virtual objects and position of the patient's virtual teeth in final position of the virtually planned orthodontic correction and/or final position of virtual artificial teeth;
- providing the simulation movements of the virtual lower jaw from the position of centric or habitual occlusion, centric relation for virtual planning of teeth position orthodontic correction taking into account visualization and control of teeth roots displacement, analysis of their biomechanics taking into account the whole tooth size, area and size of the root in the jaw bone, its proximity to the jaw bone cortical plate;
- providing the simulation movements of the virtual lower jaw from the position of centric or habitual occlusion, centric relation for virtual planning of teeth position orthodontic correction taking into account visualization, position control, sizes and form of any additional elements, fixed on the teeth surface to transfer orthodontic forces to teeth taking into account optimum biomechanics;
- providing the simulation movements of the virtual lower jaw from the position of centric or habitual occlusion, centric relation for virtual planning of orthognatic surgical operations for correction of jaw bones sizes, restoration of bones, elements of temporo-mandibular joints;
- mutually causal virtual planning of orthognatic correction of size, form and position of jaw bones, elements of temporo-mandibular joints, teeth position orthodontic correction, virtual planning of teeth size and form prosthetic correction, virtual planning of implantation in the position of centric or habitual occlusion or in virtually planned position of the centric relation taking into account the simulation movements of the virtual lower jaw, and provides designing and subsequent manufacturing of artificial dentures, orthodontic appliances and devices, appliances for bone fragments retention, guiding templates, individual implants for bone restoration, individual implants for replacement of missing teeth, repositioning and remedial splints controlling the lower jaw position;
- virtual articulator additionally includes virtual 3D curves of clinical teeth cervical zones, which are defined as crossing of the patient's teeth and the gum and set bounds to the tooth visible part, and virtual 3D curves of teeth cervical lines, which are defined as crossing of the patient's teeth and the jaw bone and set bounds to the root part in the jaw bone;
- additionally including mutually causal virtual planning of teeth position orthodontic correction and virtual planning of teeth size and form prosthetic correction from the position of centric or habitual occlusion, from the virtually set centric relation, taking into account the final teeth position at orthodontic correction and taking into account the simulation movements of the virtual lower jaw, and provides correction of the teeth gingival margin position, taking into account the changes of teeth cervical lines occurred, related to the changing of teeth roots position with respect to the jaw bone surface at orthodontic correction;
- includes providing the simulation movements of the virtual lower jaw from the position of centric or habitual occlusion, centric relation for virtual planning of teeth position orthodontic correction and virtual planning of teeth size and form prosthetic correction taking into account the size analysis of dental arches, teeth and their position, and estetigram calculation based on facial features obtained from the available virtual models of 3D and 2D face images, using the calculated regularities of visual perception of dental arches aesthetic defects;
- includes providing the simulation movements of the virtual lower jaw from the second set position to the terminal position in terms of the calculations, based on the known data about the lower jaw displacement from the first set position to the second set position and displacement from the first set position to the terminal position;
- additionally including mutually causal virtual planning of orthognatic correction of size, form and position of jaw bones, elements of temporo-mandibular joints, teeth position orthodontic correction, virtual planning of teeth size and form prosthetic correction, virtual planning of implantation and recording the planning results into the dental formula identifying the treatment plan;
- additionally including mutually causal virtual planning of orthognatic correction of size, form and position of j aw bones, elements of temporo-mandibular joints, teeth position orthodontic correction, virtual planning of teeth size and form prosthetic correction, virtual planning of implantation in the position of centric or habitual occlusion or in virtually planned position of the centric relation taking into account the simulation movements of the virtual lower jaw, and provides recording of the designed objects and also 3D model prepared for intraoral objects dental prosthetics into the individual dental-mechanical formula, identifying the initial data for designing the artificial dentures, orthodontic appliances and devices, appliances for bone fragments retention, guiding templates, individual implants for bone restoration, individual implants for replacement of missing teeth, repositioning and remedial splints controlling the lower jaw position;
- when setting the initial and terminal positions of the virtual lower jaw, the clearances between virtual surfaces of the mandible heads and virtual surfaces of the articular eminences and mandibular fossae of the patient's temporal bones are controlled and, if necessary, corrected;
- teeth position orthodontic correction in the initial position of the lower jaw is carried out taking into account the cumulative occlusion contact of the maximum area;
- additionally there is mutually causal virtual planning of orthognatic correction of size, form and position of jaw bones, elements of temporo-mandibular joints, teeth position orthodontic correction, virtual planning of teeth size and form prosthetic correction, taking into account the changes of the face virtual 3D model, conditioned by width and springiness of the face soft tissues;
- additional providing the simulation movements of the virtual lower jaw from the position of centric or habitual occlusion, centric relation to the position corresponding to the lower jaw objects position based on registration of different positions of dental arches or their fragments by means of 2D images;
- additional providing the simulation movements of the virtual lower jaw based on registration of different positions of dental arches, their fragments or reference points, fixed on teeth, by means of 3D scanning in real-time mode;

One more invention is nonvolatile machine-readable medium with recorded code containing the instructions to execute the above-mentioned method.

### BRIEF DESCRIPTION OF THE DRAWINGS

The present invention is represented in the following figures:
Fig.1A. Highlighting of mandibular head and condyle and mandibular fossa and articular eminence of the temporal bone.
Fig. IB. Virtual objects included into 3D scene, which could be used by the virtual articulator.
Fig.2. Alignment of the bite register with the upper jaw teeth.
Fig.3. Alignment of the lower jaw with the bite registers.
Fig.4. Initial position (habitual occlusion) of the lower jaw. Arrow 1 indicates the time line beginning, corresponding to the starting position. Arrow 2 indicates the sections of the mandible heads and mandibular fossae.
Fig.5. The lower jaw in the left laterotrusion position. Arrow 1 indicates the time line end, arrow 2 indicates the position of the mandible heads, arrow 3 indicates the teeth contact in this terminal position.
Fig.6. Initial position of the mandible heads and teeth before setting the terminal position.
Fig.7. The terminal position with teeth contact or with set separation is controlled by position settings of the mandible heads (arrow 1) and inter-incisal height (arrow 2).
Fig.8. Masticatory and temporal muscles attachment points are marked out.
Fig.9. The lower jaw has been displaced.
Fig.10. Displacement of the lower jaw results in changing of the distance between muscle attachment points.
Fig.11. Displacement of the lower jaw results in changing of the distance between muscle attachment points.
Fig.12. Terminal position with artificially set separation.
Fig.13. Artificial teeth compensate for the set clearance by mutual contact.
Fig.14. At arrangement the artificial tooth from the data bank is scaled to fit the corresponding tooth of the patient, aligned with it and clinical cervical zone of this tooth.
Fig.15. 2D or 3D face images provide important reference points for teeth alignment or artificial teeth arrangement.
Fig.16. 23rd tooth is missing. Adjacent teeth with the lapse of time moved to each other hiding the defect.
Fig.17. Teeth position is corrected with making a place for the artificial 23rd tooth.
Fig.18. Artificial 23rd tooth and implant to 23rd tooth root area are virtually installed.
Fig.19. Exploitation of the invention enables to use the treatment regimen reducing the treatment duration due to mutually causal designing: designing is carried out not sequentially but simultaneously, that enables to start different kinds of dental treatment at the same time.
Fig.20. Colored contact map indicates availability of contacts, subject to prevention of teeth interpenetration, and penetration depth, subject to permission of interpenetration between teeth.

### DETAILED DESCRIPTION OF THE INVENTION

The inventive method of using the dynamic virtual articulator for occlusion modeling at changing the initial position of the lower jaw, its fragments, teeth position relative to each other, position and form of artificial teeth (setting of the reconstructive bite and/or patient's teeth position orthodontic correction, and/or designing the artificial teeth form, and/or implant positioning for subsequent designing of one or more dental structures (e.g. artificial dentures, guiding templates, bite splints, orthodontic appliances) for the patient), implemented by means of a computer, including the following stages:
providing the virtual articulator, comprising the virtual 3D model of the upper jaw and dental arch and virtual 3D model of the lower jaw and dental arch, replicating the patient's upper jaw and teeth and lower jaws and teeth respectively;
providing the simulation movements of the virtual upper jaw and virtual lower jaw relative to each other for simulation modeling of dynamic occlusion at which penetrable and impenetrable contacts between teeth of the virtual upper jaw and virtual lower jaw could occur.

Provided that, providing the virtual articulator includes virtual 3D models of the temporo-mandibular joints, replicating the mandible heads, mandibular fossae and articular eminences of the temporal bones, and simulation movements of the jaws relative to each other is preceded by clinical or virtual setting of initial and terminal positions. Besides, virtual surfaces of the mandible heads and virtual surfaces of the articular eminences and mandibular fossae of the patient's temporal bones are separated.

The inventive method also includes the setting of a clearance between virtual surfaces of the teeth, subject to separation between virtual surfaces of the mandible heads and virtual surfaces of the articular eminences and mandibular fossae of the patient's temporal bones.

Due to the fact that at simulation movements the clearances between the elements of the temporo-mandibular joints and the contacts and clearances between the patient's virtual teeth are set, controlled and taken into account, the representation becomes more realistic.

Moreover, 3D treatment planning can be effected as by successive steps as simultaneously.

Besides, in particular embodiments the inventive articulator can be implemented as follows:
- integration of the used virtual models of 3D and 2D face images and 2D images of lateral and frontal teleroentgenograms; Cephalometric analysis of the teleroentgenograms presents additional important information for determination of the initial position of the centric relation between virtual upper jaw and virtual lower jaw. Facial features present additional information about midline, height and inclination of the occlusal plane;
- providing the virtual articulator with integration of 2D face (smile) images at different free positions of the lower jaw on the images. Provided that, 2D face (smile) images in virtual space are constantly bound to virtual upper teeth, and virtual models of lower teeth, virtual models of the lower jaw are temporary bound to the corresponding lower teeth or their fragments on the corresponding images. The result of the lower teeth modeling becomes visible against the background of the patient's face (smile) in cases, when, e.g. the upper teeth overlap and obstruct the view of the lower teeth;
- providing the virtual articulator with virtual models of teeth, lower jaw bone, teeth roots, soft tissues painted in the color of real objects, including the models based on used 2D images.

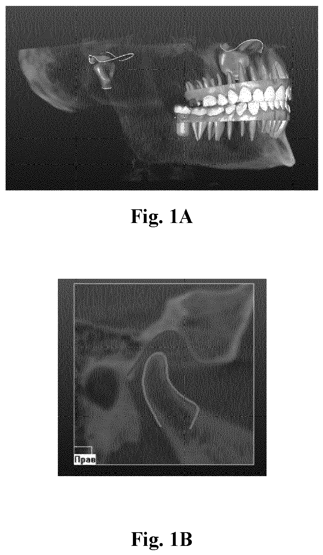

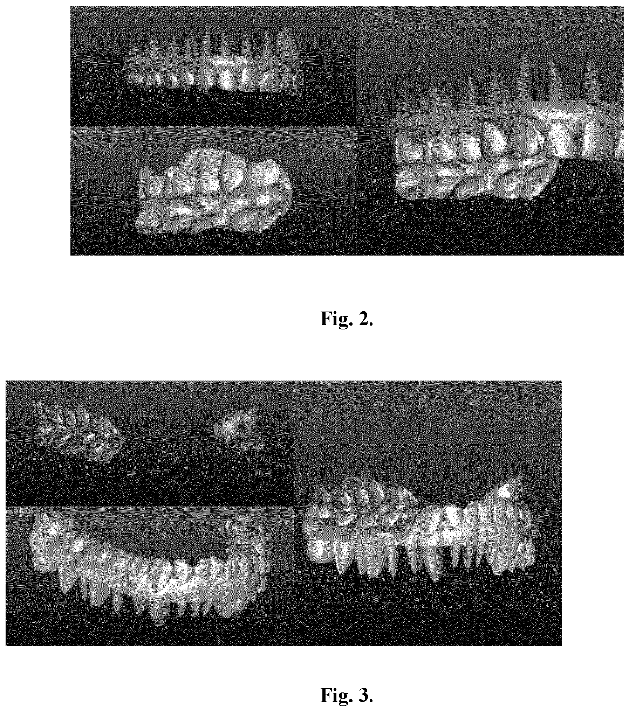

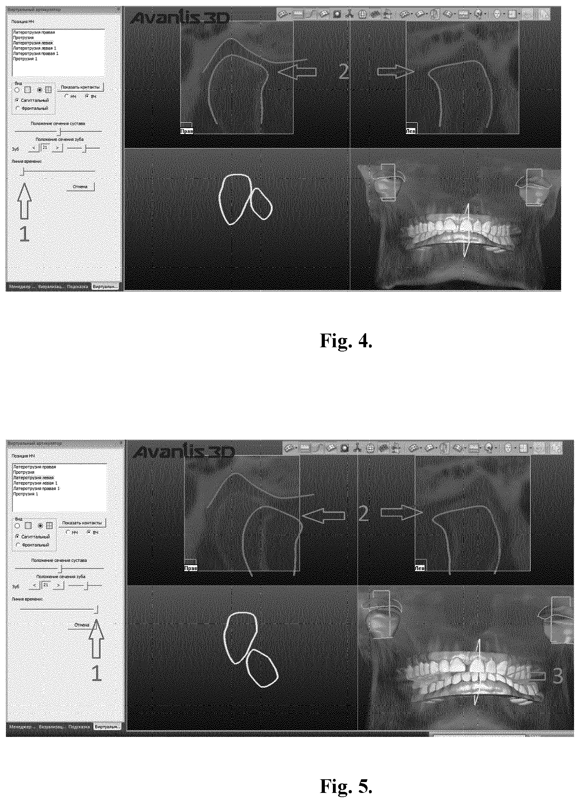

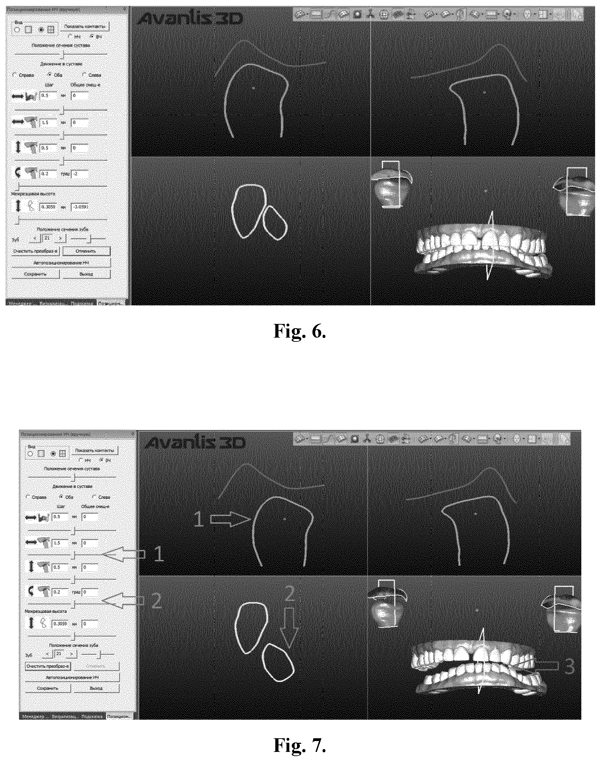

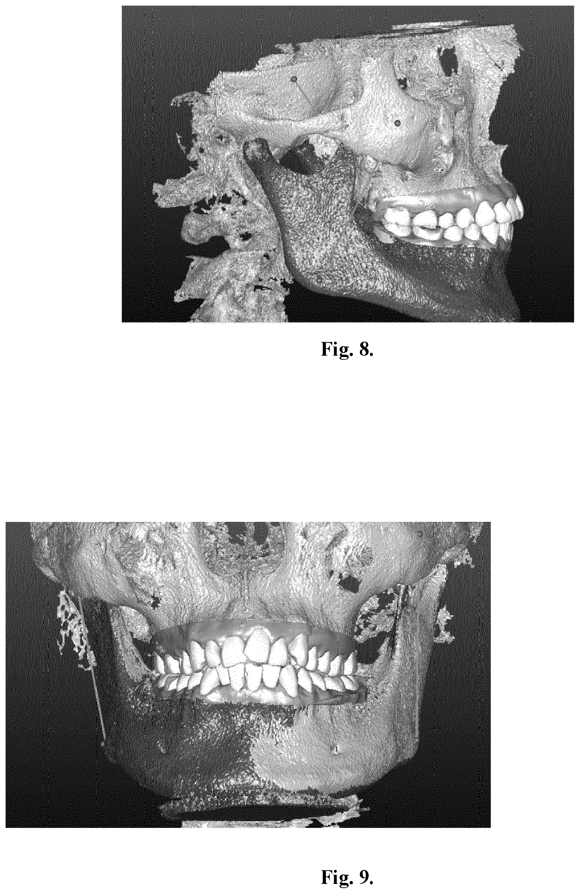

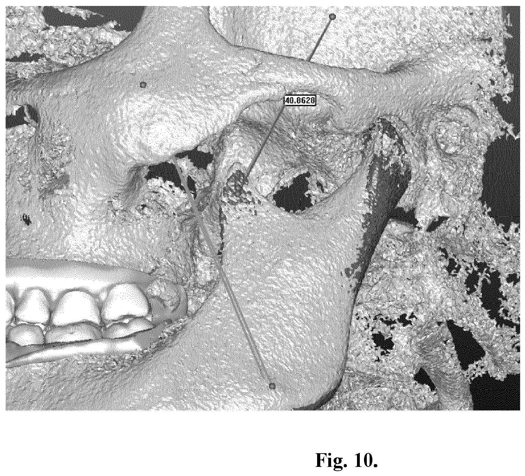

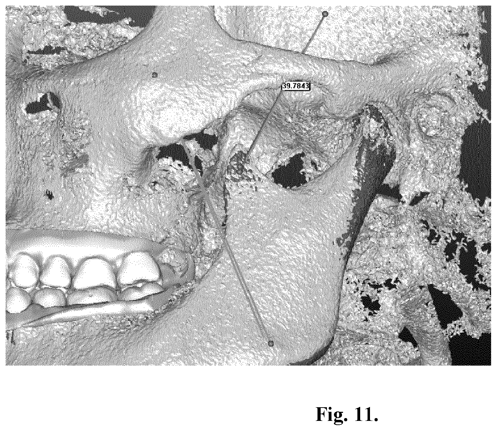

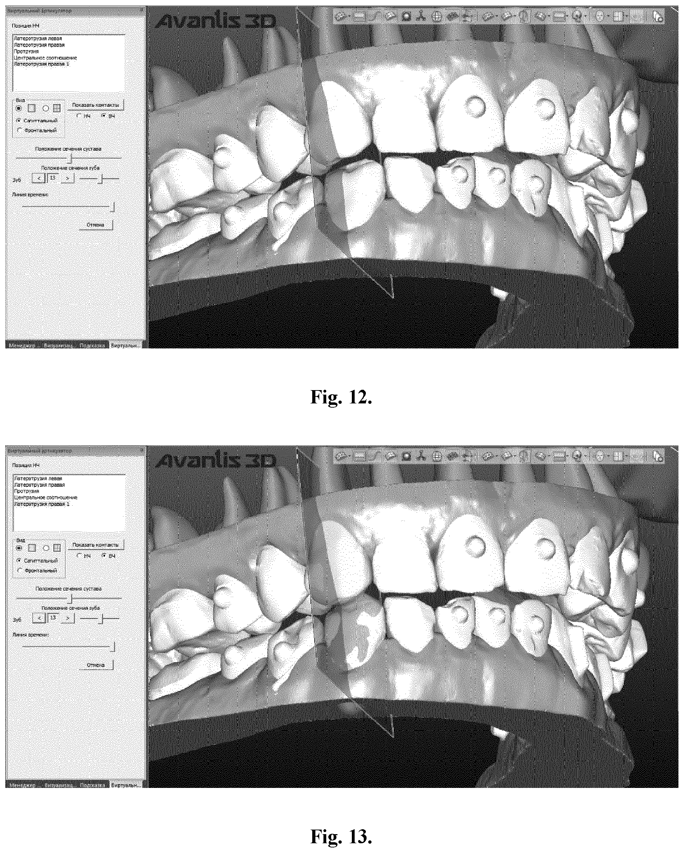

When demonstrating the modeling result, it is habitual and more natural for the patient to perceive virtual objects if they are painted the natural colors of real objects;
- providing the virtual articulator with virtual models of teeth, lower jaw, elements of the temporo-mandibular joint, obtained on the basis of direct or indirect (by means of the obtained impressions) optical, ultrasonic scanning, X-ray tomography or magnetic resonance imaging (Fig.1A, Fig.1B).
- providing the simulation movements of the virtual lower jaw based on registration of different terminal positions of the functional occlusion of dental arches (protrusion, laterotrusions, mediatrusions, etc.) by means of intraoral scanning; obtaining bite registers at these terminal positions, their subsequent scanning and sequential comparison of virtual objects of the lower dental arch, lower jaw, mandible heads (Fig.2, Fig.3);
- providing the simulation movements of the virtual lower jaw based on registration of its movements by different known methods, for example, by registration of movements of the lower jaw, lower dental arch or its fragment, one or more reference points, fixed on the lower jaw or lower teeth by means of visible or invisible electromagnetic waves, ultrasonic waves;
- providing the simulation movements of the virtual lower jaw based on anatomic-mean or individual parameters of articular and incisal guidance, lateral displacement. In all above-mentioned cases it enables the operator to evaluate and monitor position of the mandible heads with respect to the articular eminences;
- providing the simulation movements of the virtual lower jaw not from the position of centric or habitual occlusion, but from the position of centric relation. Having set the terminal position and position of the centric relation it is possible to simulate not only the movements from the initial position (habitual occlusion, centric occlusion) to the terminal position and from the initial position to the centric relation, but also to calculate mathematically and simulate the movement from the centric relation to the terminal position;
- clinical determination of the centric relation virtual position for providing the virtual articulator by direct (intraoral scanning) or indirect registration (obtaining the bite registers, their scanning and subsequent comparison of virtual registers and upper jaw teeth, and then comparison of lower jaw virtual objects, related to the lower dental arch, and lower teeth impressions on virtual registers) (Fig. 4,5).
- virtual position of centric relation for providing the virtual articulator is set by operator manually or automatically based on anatomic-mean width of articular cavities, by virtual correction of the position of the mandible heads' virtual models and related lower jaw virtual objects, and by virtual correction of inter-incisal height or vertical relations of any other selected virtual opposing teeth (Fig.6,7). Virtual correction of the patient's teeth position or virtual modeling of the artificial teeth form is carried out not iteratively, as is done in the known prior arts, but straight away taking into account the set initial and terminal position of the lower jaw;
- virtual position of centric relation for providing the virtual articulator is set by operator manually or automatically based on correction of the distance between masticatory muscles attachment points, by virtual correction of the lower jaw position (Fig.8-11). Change of muscle balance is controlled by changing the lower jaw position, since it is known in the medical field that muscle tone depends on muscle length;
- virtual position of centric relation for providing the virtual articulator is set clinically based on direct or indirect registration of the lower jaw position, at which the maximum masticatory force develops;
- direct registration includes intraoral scanning, and indirect registration includes obtaining the bite registers, their scanning and subsequent comparison of virtual registers and upper jaw teeth, and then comparison of lower jaw virtual objects, related to the lower dental arch, and lower teeth impressions on virtual registers;
- providing the simulation movements of the virtual lower jaw from the position of centric or habitual occlusion, centric relation, into the virtual terminal positions preliminarily set by operator, and thereby, changing of the patient's teeth surface at virtual planning of their direct restoration, at virtual planning of their displacement at orthodontic treatment, at virtual planning of their indirect restoration at dental prosthetics using virtual models of artificial teeth, is carried out immediately taking into account the simulation movements of the virtual lower jaw with no need for additional iterations (Fig. 12, 13).

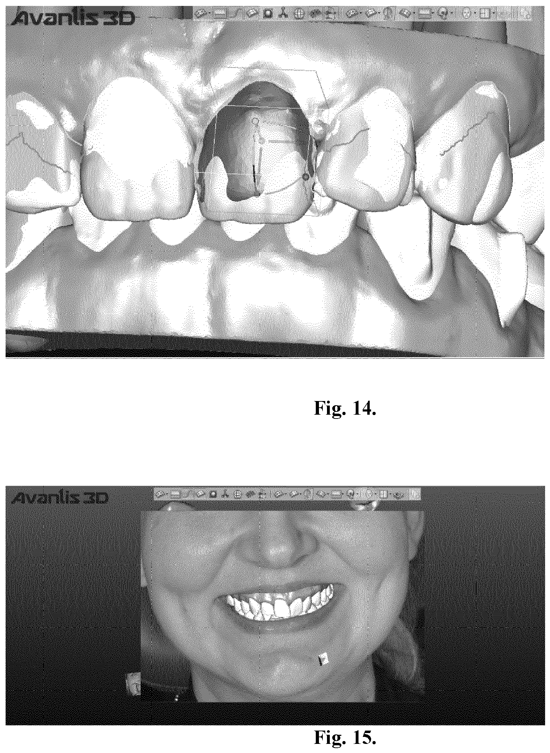

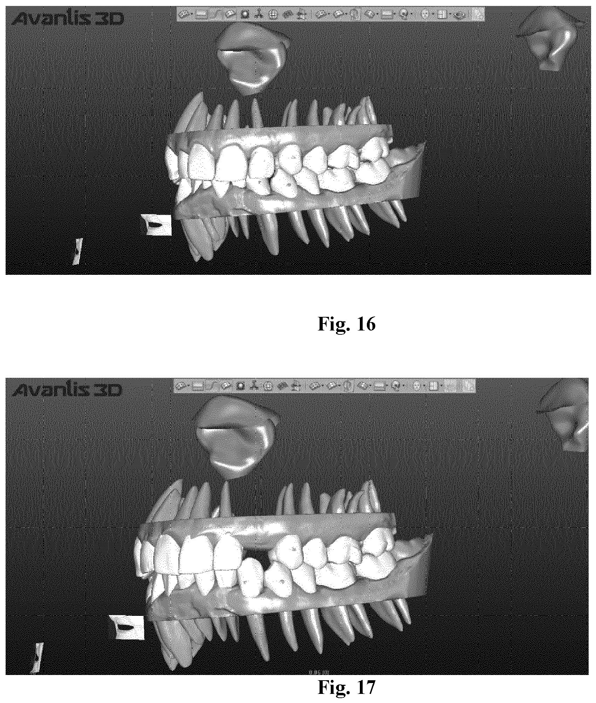

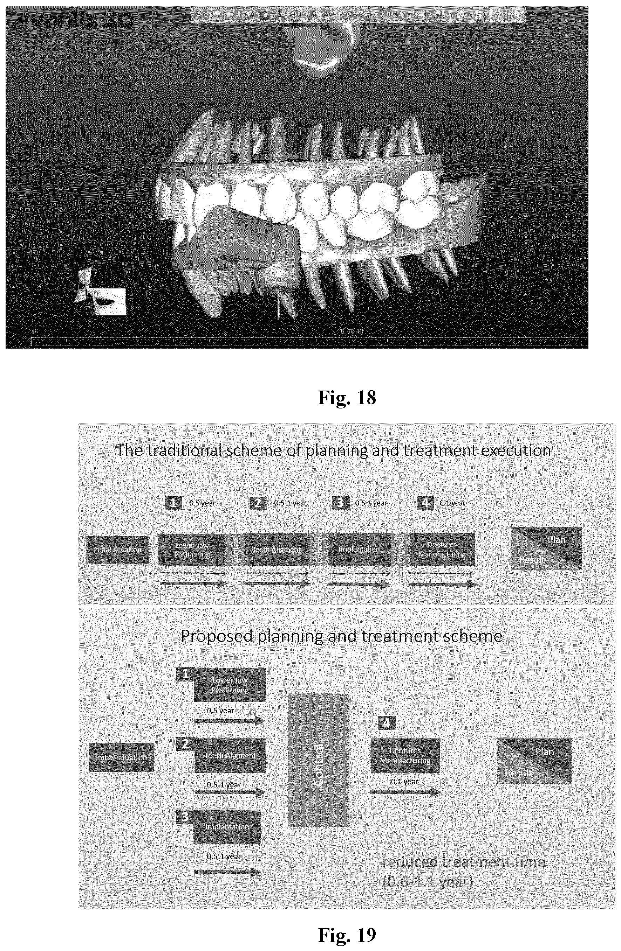

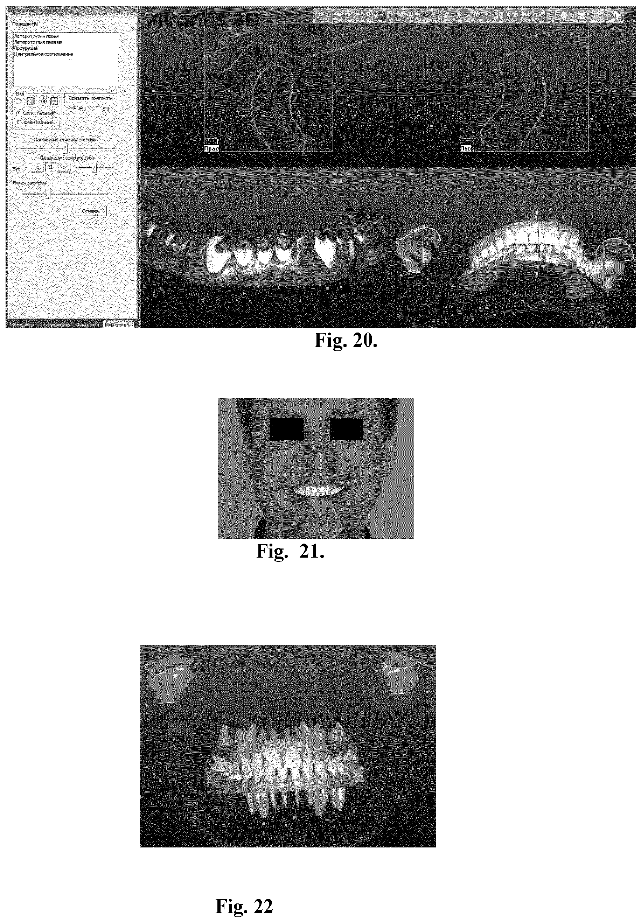

It will be appreciated that the term "virtual jaw" means not only the jaw bone by itself, but also all the objects related to it anatomically: teeth crowns and roots and also artificial teeth and implants located in the jaw, periodontal membrane, gum, maxillary sinus and incisive canal for upper jaw, heads and mandibular canal for lower jaw;
- providing the simulation movements of the virtual lower jaw from the position of centric or habitual occlusion, centric relation, when the occlusion contact points are set by changing the patient's teeth surface at virtual planning of their direct restoration, at virtual planning of their displacement at orthodontic treatment, at virtual planning of their indirect restoration at dental prosthetics using virtual models of artificial teeth;
- providing impenetrability of the patient's virtual teeth or virtual artificial teeth;
- providing penetrability of the patient's virtual teeth or virtual artificial teeth;
- virtual planning of teeth position orthodontic correction or teeth form prosthetic correction from the position of centric or habitual occlusion taking into account the simulation movements of the virtual lower jaw;
- virtual planning of teeth position orthodontic correction or teeth form prosthetic correction from the virtually set centric relation, taking into account the simulation movements of the virtual lower jaw;
- mutually causal virtual planning of teeth position orthodontic correction and virtual planning of teeth form and size prosthetic correction from the position of centric or habitual occlusion, from the virtually set centric relation, taking into account the final teeth position at orthodontic correction and taking into account the simulation movements of the virtual lower jaw. Mutual causality of the virtual planning of teeth position orthodontic correction and teeth form prosthetic correction originates from the fact that artificial teeth from the data bank for prosthetic correction are scaled taking into account sizes and forms of the patient's available virtual teeth, symmetrical tooth and/or proportionally to the available teeth in case of missing one or several teeth, aligned with the patient's virtual teeth taking into account the maximum possible alignment of surfaces, long axes of the artificial tooth and the patient's tooth, and binding of the artificial tooth to clinical or cervical lines of the patient's virtual tooth or to their projection if the tooth is missing. Alignment of the virtual artificial teeth with the patient's virtual teeth is effected either in the initial or final position of the patient's teeth at virtual planning of their alignment. Therefore, the virtual planning of teeth alignment itself is carried out taking into account the size and form of the artificial teeth and the correction of the artificial teeth sizes and form is done taking into account the alignment of the patient's virtual teeth, and all this is performed with consideration to the initially set position of the virtual lower jaw, with consideration to its virtual simulation movements and with consideration to the facial features, obtained from 3D or 2D images, included into the virtual objects (Fig. 14, 15). It enables to reduce time expenditures for virtual modeling due to the tool implementing the method of mutually causal and simultaneous virtual modeling of the artificial teeth form and position and the patient's teeth position taking into account the virtually set initial and terminal position of the lower jaw;
- mutually causal virtual planning of implantation taking into account the jaw virtual objects and position of the patient's virtual teeth in final position of the virtually planned orthodontic correction and/or final position of virtual artificial teeth (Fig. 16-18); Mutually causal virtual planning of teeth position orthodontic correction, virtual planning of teeth sizes and form prosthetic correction, virtual planning of implantation in the position of centric or habitual occlusion or in the virtually planned position of the centric relation, taking into account the simulation movements of the virtual lower jaw into the virtually planned terminal positions (protrusion, laterotrusion, etc.) makes it possible to start all the kinds of the planned treatment or their separate combinations simultaneously: setting the reconstructive position of the lower jaw, teeth alignment, teeth form prosthetic correction, implantation, that considerably reduces possible total treatment time (Fig. 19);
- providing the simulation movements of the virtual lower jaw from the position of centric or habitual occlusion, centric relation for virtual planning of teeth position orthodontic correction taking into account visualization and control of teeth roots displacement, analysis of their biomechanics taking into account the whole tooth size, area and size of the root in the jaw bone, its proximity to the jaw bone cortical plate (surface) (Fig. 16,17). It enables to increase the probability of reaching the virtually planned result and decrease the probability of complications during treatment.
- providing the simulation movements of the virtual lower jaw from the position of centric or habitual occlusion, centric relation for virtual planning of teeth position orthodontic correction taking into account visualization, position control, sizes and form of any additional elements (attachments, braces, etc.) fixed on the teeth surface to transfer orthodontic forces to teeth taking into account optimum biomechanics (Fig.20);
- providing the simulation movements of the virtual lower jaw from the position of centric or habitual occlusion, centric relation for virtual planning of orthognatic surgical operations for correction of jaw bones sizes, restoration of bones, elements of temporo-mandibular joints;
- mutually causal virtual planning of orthognatic correction of size, form and position of jaw bones, elements of temporo-mandibular joints, teeth position orthodontic correction, virtual planning of teeth size and form prosthetic correction, virtual planning of implantation in the position of centric or habitual occlusion or in virtually planned position of the centric relation taking into account the simulation movements of the virtual lower jaw, and provides designing and subsequent manufacturing of artificial dentures, orthodontic appliances and devices, appliances for bone fragments retention, guiding templates, individual implants for bone restoration, individual implants for replacement of missing teeth, repositioning and remedial splints controlling the lower jaw position;
- providing the virtual articulator by including the virtual 3D curves of clinical teeth cervical zones, which are defined as crossing of the patient's teeth and the gum and set bounds to the tooth visible part, and virtual 3D curves of teeth cervical lines, which are defined as crossing of the patient's teeth and the jaw bone and set bounds to the root part in the jaw bone;
- mutually causal virtual planning of teeth position orthodontic correction and virtual planning of teeth size and form prosthetic correction from the position of centric or habitual occlusion, from the virtually set centric relation, taking into account the final teeth position at orthodontic correction and taking into account the simulation movements of the virtual lower jaw, and provides the artificial correction of the teeth gingival margin (clinical cervical zone) position by operator, related to the changing of teeth roots position with respect to the jaw bone surface at orthodontic correction; It enables to increase representation objectivity of actual processes at their virtual modeling;
- providing the simulation movements of the virtual lower jaw from the position of centric or habitual occlusion, centric relation for virtual planning of teeth position orthodontic correction and virtual planning of teeth size and form prosthetic correction taking into account the size analysis of dental arches, teeth and their position, and estetigram calculation based on facial features (face midline, interpupillary line, mouth slit size at smile) obtained from the available virtual models of 3D and 2D face images, using the calculated regularities of visual perception of dental arches aesthetic defects; Therefore, artificial intelligence elements are involved in the process of virtual modeling of artificial teeth form and position from the perspective of their possible visual perception;
- providing the simulation movements of the virtual lower jaw from the second initial position (e.g. centric relation) to the terminal position in terms of the calculations, based on the known data about the lower jaw displacement from the first initial position (e.g. centric or habitual occlusion) to the second initial position (e.g. centric relation) and displacement from the first initial position to the terminal position;
- mutually causal virtual planning of orthognatic correction of size, form and position of jaw bones, elements of temporo-mandibular joints, teeth position orthodontic correction, virtual planning of teeth size and form prosthetic correction, virtual planning of implantation and recording the planning results into the dental formula identifying the treatment plan;
- additionally including mutually causal virtual planning of orthognatic correction of size, form and position of jaw bones, elements of temporo-mandibular joints, teeth position orthodontic correction, virtual planning of teeth size and form prosthetic correction, virtual planning of implantation in the position of centric or habitual occlusion or in virtually planned position of the centric relation taking into account the simulation movements of the virtual lower jaw, and provides recording of the designed objects and also 3D model prepared for intraoral objects dental prosthetics into the individual dental-mechanical formula, identifying the initial data for designing the artificial dentures, orthodontic appliances and devices, appliances for bone fragments retention, guiding templates, individual implants for bone restoration, individual implants for replacement of missing teeth, repositioning and remedial splints controlling the lower jaw position;
- when setting the initial and terminal positions of the virtual lower jaw, the clearances between virtual surfaces of the mandible heads and virtual surfaces of the articular eminences and mandibular fossae of the patient's temporal bones are controlled and, if necessary, corrected;
- teeth position orthodontic correction in the initial position of the lower jaw is carried out taking into account the cumulative occlusion contact of the maximum area;
- mutually causal virtual planning of orthognatic correction of size, form and position of jaw bones, elements of temporo-mandibular joints, teeth position orthodontic correction, virtual planning of teeth size and form prosthetic correction, taking into account the changes of the face virtual 3D model, conditioned by width and springiness of the face soft tissues;
- providing the simulation movements of the virtual lower jaw from the position of centric or habitual occlusion, centric relation to the position corresponding to the lower jaw objects position based on registration of different positions of dental arches or their fragments by means of 2D images;
- providing the simulation movements of the virtual lower jaw based on registration of different positions of dental arches, their fragments or reference points, fixed on teeth, by means of 3D scanning in real-time mode;

Besides, one embodiment of the invention is a nonvolatile machine-readable medium with recorded code containing the instructions to execute the above-mentioned method.

### Example.

Patient D., cross bite, displacement of upper and lower dental arches midline, increased dental attrition, complete destruction of 45 tooth crown, partial defects of 46, 47 teeth crowns, dental bridge supported on 35-37 teeth (Fig. 21. 3D scene includes the photo of the smiling patient).

Initial 3D scene (Fig. 22) consists of CT data, scans of dental arches and gum of the upper and lower jaws, mandible heads and mandibular fossae of the temporal bones.

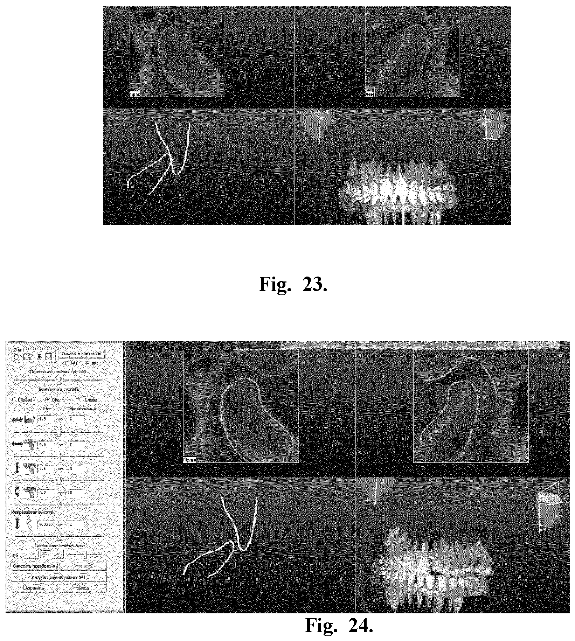

Cross bite was caused by the fact that the lower jaw in the habitual occlusion position had been forcedly displaced to the left due to the bite peculiarities. In this habitual occlusion position the mandible heads are displaced distally and are asymmetrical (Fig. 23. The mandible heads are asymmetrically located in the mandibular fossae due to displacement of the lower jaw and are displaced distally due to decrease of the occlusal vertical dimension caused by dental attrition).

The centric relation position is virtually set and saved in the program. This position is ensured by displacement of the lower jaw while controlling the relation between the elements of temporo-mandibular joints and dental bite (Fig. 24. The controllable clearance between the mandible heads and mandibular fossae is set. When the lower jaw is displaced, the midlines are aligned).

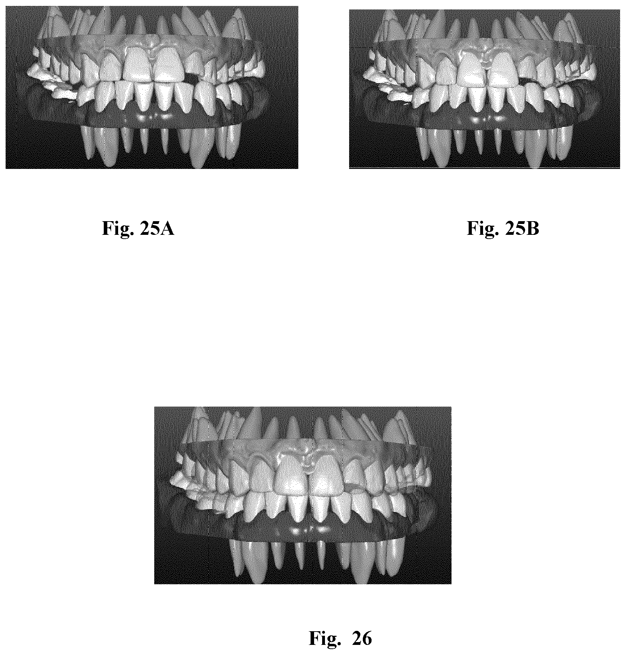

Preliminary scenario of teeth alignment is prepared. It involves expansion of the upper jaw dental arch due to labial protrusion of teeth 22-27, decrease of labial inclination of the upper anterior teeth 12-21 with coverage of existing tremas, oral dislocation of the lower anterior teeth 43-34 with elimination of the cross bite, and alignment of midlines (Fig. 25A (position of the virtual teeth before alignment), Fig. 25B (position of the virtual teeth after alignment. The alignment scenario is prepared in the virtually set position of the lower jaw centric relation))

Artificial teeth from the data bank are arranged and adjusted to clinical cervical zones in the final position of the patient's aligned virtual teeth. The smile design is performed by correcting their position and form (Fig. 26. Artificial teeth are installed into the projection of the final position of the patient/s virtual natural teeth in conformity with clinical cervical zones of these teeth).

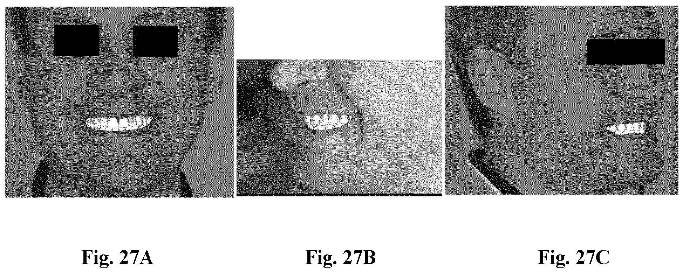

Provided that, teeth form is evaluated at smile on the background of facial features (Fig.27 A-C - photos of the smiling patient, integrated into 3D scene, enable to evaluate the smile design).

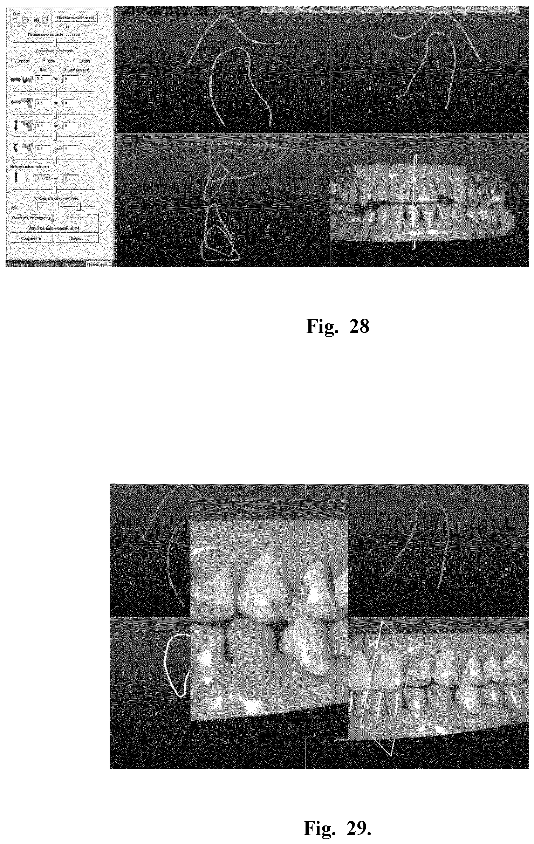

Teeth relation is checked in the virtual articulator based on the simulation movement of the virtual lower jaw from the virtually set position of centric relation to the virtually set position of the left laterotrusion at the controllable clearances between the mandible heads and mandibular fossae and the controllable clearance between left canine teeth (Fig. 28 - virtual position of the left laterotrusion is set by forward displacement of the right mandible head, lateral displacement of both heads and rotation of the lower jaw around the axis between the mandible heads, controlling the required clearance between left canine teeth (canine guidance). Then this clearance is packed owing to height of the artificial tooth, Fig. 29 - the artificial teeth contacts are controlled, and their form is correspondingly corrected at simulation movements of the virtual lower jaw).

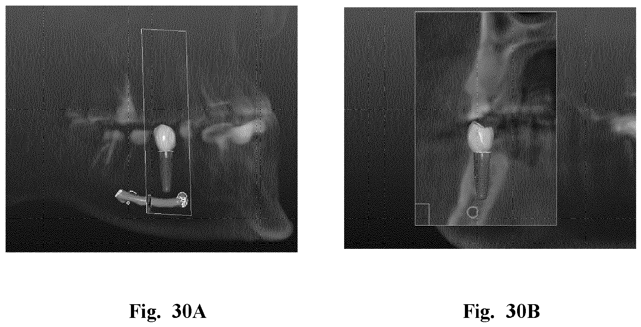

Finally, the implant was virtually installed in the root area of 45 tooth, which was planned for extraction, with consideration to the position of the future artificial 45 tooth, bone volume in this area, location of roots of adjacent teeth and mandibular canal (Fig. 30 A-B - virtual implant is installed in the cross-section plane in conformity with the axis of the future artificial tooth and its crown position).

Based on the implant set position the guiding template is designed, and the implant is installed into the patient's jaw. Based on the set scenario of teeth displacement by means of 3D prototyping the aligners are manufactured and teeth are aligned. All kinds of treatment are started simultaneously. While the implant is taken (osteointegrated), the patient's teeth are aligned.

On completing the alignment, the patient's teeth are prepared, scanned and, in accordance with the smile design developed before, the temporary and permanent crowns are manufactured and fix the patient's lower jaw in the original virtually set position of centric relation.

## Claims

1. A method for using a dynamic virtual articulator for occlusion modeling when changing an initial position of lower jaw or its fragments, teeth positions relative to each other, position and shape of artificial teeth, the method is implemented using a computer and comprises the following stages:
providing a virtual articulator comprising a virtual three-dimensional model of an upper jaw and dental arch, and a virtual three-dimensional model of a lower jaw and dental arch, replicating the upper jaw and upper teeth, and the lower jaw and lower teeth of a patient, respectively, and
providing simulation movements of virtual upper jaw and virtual lower jaw relative to each other for simulation modeling of dynamic occlusion, wherein during such movements penetrable or impenetrable contacts between teeth of the virtual upper jaw and virtual lower jaw occur;
**characterized in that**
providing the virtual articulator includes providing virtual three-dimensional models of temporo-mandibular joints, replicating mandible heads, mandibular fossa and articular eminences of temporal bones of the patient, and simulation movements of the virtual jaws relative to each other are preceded by clinical or virtual setting of initial and terminal positions, and
virtual surfaces of the mandible heads and virtual surfaces of the articular eminences and mandibular fossa of the patient's temporal bones are separated.

2. The method according to claim 1, **characterized in that** the virtual articulator further includes virtual models of 3D and 2D images of face and 2D images of side and front telerentgenograms.

3. The method according to claim 1, **characterized in that** virtual models of teeth, lower jaw, elements of the temporo-mandibular joint are obtained on a basis of direct or indirect scanning.

4. The method according to claim 1, **characterized in that** it includes providing simulation movements of the virtual lower jaw based on registration of different terminal positions of dental arches by means of intraoral scanning; and/or obtaining bite registers at specified different terminal positions, their subsequent scanning and sequential comparison of virtual objects of the lower dental arch, lower jaw, mandible heads.

5. The method according to claim 1, **characterized in that** it includes providing simulation movements of the virtual lower jaw from a centric relation position.

6. The method according to claim 5 , **characterized in that** the centric relation position is set clinically using direct or indirect registration.

7. The method according to claim 5 , **characterized in that** the centric relation position is set by an operator manually or automatically based on anatomic-mean width of articular cavities, by virtual correction of position of mandible heads' virtual models and related lower jaw virtual objects, and by virtual correction of inter-incisal height or vertical relations of any other selected virtual opposing teeth.

8. The method according to claim 1, **characterized in that** it includes providing simulation movements of the virtual lower jaw from a position of centric or habitual occlusion, centric relation, when occlusion contact points are set by changing patient's teeth surface at virtual planning of their direct restoration, at virtual planning of their displacement at orthodontic treatment, at virtual planning of their indirect restoration at dental prosthetics using virtual models of artificial teeth.

9. The method according to claim 1, further comprising mutually causal virtual planning of implantation taking into account jaw virtual objects and position of patient's virtual teeth in final position of virtually planned orthodontic correction and/or final position of virtual artificial teeth.

10. The method according to claim 1, **characterized in that** it includes providing simulation movements of virtual lower jaw from position of centric or habitual occlusion, centric relation for virtual planning of teeth position orthodontic correction taking into account visualization and control of teeth roots displacement, analysis of their biomechanics taking into account a whole tooth size, area and size of the root in a jaw bone, its proximity to a jaw bone cortical plate.

11. The method according to claim 1, **characterized in that** it includes providing simulation movements of virtual lower jaw from position of centric or habitual occlusion, centric relation for virtual planning of teeth position orthodontic correction taking into account visualization, position control, sizes and form of any additional elements, fixed on teeth surface to transfer orthodontic forces to teeth taking into account optimum biomechanics.

12. The method according to claim 1, **characterized in that** it includes providing simulation movements of virtual lower jaw from position of centric or habitual occlusion, centric relation for virtual planning of orthognatic surgical operations for correction of jaw bones sizes, restoration of bones, elements of temporo-mandibular joints.

13. The method according to claim 1, further comprising mutually causal virtual planning of orthognatic correction of size, form and position of jaw bones, elements of temporo-mandibular joints, teeth position orthodontic correction, virtual planning of teeth size and form prosthetic correction, virtual planning of implantation in position of centric or habitual occlusion or in virtually planned position of the centric relation taking into account simulation movements of virtual lower jaw, and provides designing and subsequent manufacturing of artificial dentures, orthodontic appliances and devices, appliances for bone fragments retention, guiding templates, individual implants for bone restoration, individual implants for replacement of missing teeth, repositioning and remedial splints controlling lower jaw position.

14. The method according to claim 1, further comprising providing simulation movements of virtual lower jaw from a second set position to a terminal position by means of calculations, based on known data about lower jaw displacement from a first set position to the second set position and displacement from the first set position to the terminal position.

15. A non-volatile machine-readable medium with a recorded code containing instructions to execute the method according to claim 1.

## Patentansprüche

1. Verfahren zur Verwendung eines dynamischen virtuellen Artikulators zum Modellieren einer Okklusion, wenn eine Anfangsposition eines Unterkiefers oder seiner Fragmente, Zahnpositionen relativ zueinander sowie eine Position und eine Form künstlicher Zähne geändert werden, wobei das Verfahren unter Verwendung eines Computers implementiert wird und die folgenden Schritte umfasst:
Bereitstellen eines virtuellen Artikulators, welcher ein virtuelles dreidimensionales Modell eines Oberkiefers und eines Zahnbogens und ein virtuelles dreidimensionales Modell eines Unterkiefers und eines Zahnbogens umfasst, Replizieren des Oberkiefers und oberer Zähne bzw. des Unterkiefers und unterer Zähne eines Patienten, und
Bereitstellen von Simulationsbewegungen eines virtuellen Oberkiefers und eines virtuellen Unterkiefers relativ zueinander zum Simulationsmodellieren einer dynamischen Okklusion, wobei während solcher Bewegungen durchdringbare oder undurchdringbare Kontakte zwischen Zähnen des virtuellen Oberkiefers und des virtuellen Unterkiefers auftreten;
**dadurch gekennzeichnet, dass**
das Bereitstellen des virtuellen Artikulators ein Bereitstellen virtueller dreidimensionaler Modelle von Kiefergelenken, ein Nachbilden von Unterkieferköpfen, einer Unterkiefer-Fossa und Gelenkhöckern von Schläfenbeinen des Patienten umfasst, und Simulationsbewegungen der virtuellen Kiefer relativ zueinander eine klinische oder virtuelle Festlegung von Anfangs- und Endpositionen vorausgeht, und
virtuelle Flächen der Unterkieferköpfe und virtuelle Flächen der Gelenkhöcker und der Unterkiefer-Fossa der Schläfenbeine des Patienten separiert werden.

2. Verfahren nach Anspruch 1, **dadurch gekennzeichnet, dass** der virtuelle Artikulator ferner virtuelle Modelle von 3D- und 2D-Bildern eines Gesichts und von 2D-Bildern von seitlichen und vorderen Telerentgenogramen umfasst.

3. Verfahren nach Anspruch 1, **dadurch gekennzeichnet, dass** virtuelle Modelle von Zähnen, eines Unterkiefers und von Elementen des Kiefergelenks auf einer Grundlage eines direkten oder indirekten Scannens erhalten werden.

4. Verfahren nach Anspruch 1, **dadurch gekennzeichnet, dass** es ein Bereitstellen von Simulationsbewegungen des virtuellen Unterkiefers auf Grundlage einer Registrierung verschiedener Endpositionen von Zahnbögen mittels Intraoral-Scannen; und/oder ein Erhalten von Bissregistraten an festgelegten verschiedenen Endpositionen, deren anschließendes Scannen und einen sequenziellen Vergleich virtueller Objekte des unteren Zahnbogens, des Unterkiefers und der Unterkieferköpfe umfasst.

5. Verfahren nach Anspruch 1, **dadurch gekennzeichnet, dass** es ein Bereitstellen von Simulationsbewegungen des virtuellen Unterkiefers von einer zentrischen Bezugsposition umfasst.

6. Verfahren nach Anspruch 5, **dadurch gekennzeichnet, dass** die zentrische Bezugsposition klinisch unter Verwendung direkter oder indirekter Registrierung festgelegt wird.

7. Verfahren nach Anspruch 5, **dadurch gekennzeichnet, dass** die zentrische Bezugsposition durch einen Bediener manuell oder automatisch auf Grundlage einer anatomisch mittleren Breite von Gelenkhöhlen, durch virtuelle Korrektur einer Position virtueller Modelle von Kieferköpfen und zugehöriger virtueller Objekte eines Unterkiefers, und durch virtuelle Korrektur einer inter-inzisalen Höhe oder vertikaler Beziehungen beliebiger anderer ausgewählter gegenüberliegender virtueller Zähne festgelegt wird.

8. Verfahren nach Anspruch 1, **dadurch gekennzeichnet, dass** es ein Bereitstellen von Simulationsbewegungen des virtuellen Unterkiefers aus einer Position einer zentrischen oder habituellen Okklusion, einer zentrischen Relation, umfasst, wenn Okklusionskontaktstellen festgelegt werden, indem die Zahnfläche eines Patienten bei einer virtuellen Planung ihrer direkten Wiederherstellung, bei einer virtuellen Planung ihrer Verlagerung bei orthodontischer Behandlung, bei einer virtuellen Planung ihrer indirekten Wiederherstellung an Zahnprothesen unter Verwendung virtueller Modelle künstlicher Zähne geändert wird.

9. Verfahren nach Anspruch 1, ferner umfassend ein gegenseitiges kausales virtuelles Planen einer Implantation unter Berücksichtigung virtueller Kieferobjekte und einer Position virtueller Zähne eines Patienten in einer finalen Position einer virtuell geplanten orthodontischen Korrektur und/oder einer finalen Position virtueller künstlicher Zähne.

10. Verfahren nach Anspruch 1, **dadurch gekennzeichnet, dass** es ein Bereitstellen von Simulationsbewegungen eines virtuellen Unterkiefers aus einer Position einer zentrischen oder habituellen Okklusion, einer zentrischen Relation für eine virtuelle Planung einer orthodontischen Korrektur von Zahnpositionen unter Berücksichtigung einer Visualisierung und einer Steuerung einer Verlagerung von Zahnwurzeln, einer Analyse ihrer Biomechanik unter Berücksichtigung einer gesamten Zahngröße, einer Fläche und einer Größe der Wurzel in einem Kieferknochen, seiner Nähe zu einer Kieferknochen-Kortikalplatte umfasst.

11. Verfahren nach Anspruch 1, **dadurch gekennzeichnet, dass** es ein Bereitstellen von Simulationsbewegungen eines virtuellen Unterkiefers aus einer Position einer zentrischen oder habituellen Okklusion, einer zentrischen Relation für eine virtuelle Planung einer orthodontischen Korrektur von Zahnpositionen unter Berücksichtigung einer Visualisierung, einer Positionssteuerung, von Größen und einer Form beliebiger zusätzlicher Elemente umfasst, welche an einer Zahnfläche fixiert sind, um orthodontische Kräfte unter Berücksichtigung einer optimalen Biomechanik auf Zähne zu übertragen.

12. Verfahren nach Anspruch 1, **dadurch gekennzeichnet, dass** es ein Bereitstellen von Simulationsbewegungen eines virtuellen Unterkiefers aus einer Position einer zentrischen oder habituellen Okklusion, einer zentrischen Relation für eine virtuelle Planung orthognatischer chirurgischer Operationen für eine Korrektur von Kieferknochenabmessungen, eine Wiederherstellung von Knochen und von Elementen von Kiefergelenken umfasst.

13. Verfahren nach Anspruch 1, ferner umfassend ein gegenseitiges kausales virtuelles Planen einer orthognatischen Korrektur einer Größe, einer Form und einer Position von Kieferknochen, von Elementen von Kiefergelenken, einer orthodonitischen Korrektur einer Zahnposition, ein virtuelles Planen einer prothetischen Korrektur einer Zahngröße und einer Zahnform, ein virtuelles Planen einer Implantation in einer Position einer zentrischen oder habituellen Okklusion oder in einer virtuell geplanten Position der zentrischen Relation unter Berücksichtigung von Simulationsbewegungen des virtuellen Unterkiefers, und welches ein Gestalten und anschließendes Herstellen künstlichen Zahnersatzes, orthodontischer Mittel und Vorrichtungen, von Mitteln für eine Erhaltung von Knochenfragmenten, von Führungsschablonen, individueller Implantate zur Knochenwiederherstellung, individueller Implantate zum Ersetzen fehlender Zähne, ein Repositionieren und Hilfsschienen zur Steuerung der Unterkieferposition bereitstellt.

14. Verfahren nach Anspruch 1, ferner umfassend ein Bereitstellen von Simulationsbewegungen eines virtuellen Unterkiefers von einer zweiten festgelegten Position zu einer Endposition mittels Berechnungen, auf Grundlage bekannter Daten über eine Verlagerung eines Unterkiefers von einer ersten festgelegten Position zu der zweiten festgelegten Position und einer Verlagerung von der ersten festgelegten Position zu der Endposition.

15. Nichtflüchtiges maschinenlesbares Medium mit einem aufgezeichneten Code, welcher Anweisungen enthält, um das Verfahren nach Anspruch 1 auszuführen.

## Revendications

1. Procédé d'utilisation d'un articulateur virtuel dynamique pour la modélisation d'une occlusion lorsqu'une position initiale de la mâchoire inférieure ou de ses fragments, les positions de dents les unes par rapport aux autres, la position et la forme de dents artificielles sont changées, le procédé étant mis en œuvre en utilisant un ordinateur et comprenant les étapes suivantes :
mettre à disposition un articulateur virtuel comprenant un modèle tridimensionnel d'une mâchoire supérieure et d'un arc dentaire, et un modèle tridimensionnel virtuel d'une mâchoire inférieure et d'un arc dentaire, reproduisant la mâchoire supérieure et les dents supérieures et respectivement la mâchoire inférieure et les dents inférieures d'un patient, et
mettre à disposition des mouvements de simulation de la mâchoire supérieure virtuelle et de la mâchoire inférieure virtuelle l'une par rapport à l'autre pour simuler une modélisation d'occlusion dynamique, pendant de tels mouvements, des contacts de pénétration ou de non-pénétration entre des dents de la mâchoire supérieure virtuelle et de la mâchoire inférieure virtuelle se produisant ;
**caractérisé en ce que**
mettre à disposition l'articulateur virtuel comprend mettre à disposition des modèles tridimensionnels virtuels d'articulations temporo-mandibulaires,
reproduire des têtes de mandibule, des fosses mandibulaires et des éminences articulaires d'os temporaux du patient, et des mouvements de simulation des mâchoires virtuelles l'une par rapport à l'autre sont précédés par un réglage clinique ou virtuel de positions de début et de fin, et
des surfaces virtuelles des têtes de mandibule et des surfaces virtuelles des éminences articulaires et des fosses mandibulaires des os temporaux du patient sont séparées.

2. Procédé selon la revendication 1, **caractérisé en ce que** l'articulateur virtuel comprend en outre des modèles virtuels d'images 3D et 2D de visages et des images 2D de telerentgenogrammes de côté et de face.

3. Procédé selon la revendication 1, **caractérisé en ce que** des modèles virtuels de dents, de mâchoire inférieure, d'éléments de l'articulation temporo-mandibulaire sont obtenus sur une base de balayage direct ou indirect.

4. Procédé selon la revendication 1, **caractérisé en ce qu'**il comprend mettre à disposition des mouvements de simulation de la mâchoire inférieure virtuelle sur la base d'un enregistrement de différentes positions de fin d'arcs dentaires moyennant un balayage intraoral ; et/ou obtenir des registres de mordu à des positions de fin différentes spécifiées, leur balayage subséquent et la comparaison séquentielle d'objets virtuels de l'arc dentaire inférieur, de la mâchoire inférieure, des têtes de mandibule.

5. Procédé selon la revendication 1, **caractérisé en ce qu'**il comprend mettre à disposition des mouvements de simulation de la mâchoire inférieure virtuelle à partir d'une position de relation centrique.

6. Procédé selon la revendication 5, **caractérisé en ce que** la position de relation centrique est réglée cliniquement en utilisant un enregistrement direct ou indirect.

7. Procédé selon la revendication 5, **caractérisé en ce que** la position de relation centrique est réglée manuellement par un opérateur ou automatiquement sur la base d'une largeur anatomiquement moyenne de cavités articulaires, par correction virtuelle de la position de modèles virtuels de tête de mandibule et d'objets virtuels de mâchoire inférieure correspondants et par correction virtuelle de rapports de hauteur inter-incisale ou verticale de toutes autres dents virtuelles opposées choisies.

8. Procédé selon la revendication 1, **caractérisé en ce qu'**il comprend mettre à disposition des mouvements de simulation de la mâchoire inférieure virtuelle à partir d'une position d'occlusion centrée ou habituelle, relation centrée, lorsque des points de contact d'occlusion sont posés en changeant la surface des dents du patient au planning virtuel de leur restauration direct, au planning virtuel de leur déplacement lors d'un traitement orthodontique, au planning virtuel de leur restauration indirecte lors de traitements prothétiques dentaux utilisant des modèles virtuels de dents artificielles.

9. Procédé selon la revendication 1, comprenant en outre planning virtuel causal commun d'implantation tenant compte d'objets virtuels de mâchoire et de la position de dents virtuelles du patient dans la position finale d'une correction orthodontique planifiée virtuellement et/ou la position finale de dents artificielles virtuelles.

10. Procédé selon la revendication 1, **caractérisé en ce qu'**il comprend mettre à disposition des mouvements de simulation de la mâchoire inférieure virtuelle à partir d'une position d'occlusion centrée ou habituelle, relation centrée, pour le planning virtuel d'une correction orthodontique de la position de dents tenant compte de la visualisation et le contrôle de déplacement de racines de dents, analyse de leur biomécanique tenant compte de la taille d'une dent entière, de la place et taille de la racine dans un os de mâchoire, sa proximité d'une plaque corticale d'os de mâchoire.

11. Procédé selon la revendication 1, **caractérisé en ce qu'**il comprend mettre à disposition des mouvements de simulation de la mâchoire inférieure virtuelle à partir d'une position d'occlusion centrée ou habituelle, relation centrée, pour le planning virtuel d'une correction orthodontique de la position de dents tenant compte de la visualisation, le contrôle de position, les tailles et forme de tout élément supplémentaire fixé sur la surface de dent pour transférer des efforts orthodontiques aux dents en tenant compte des biomécaniques optimums.

12. Procédé selon la revendication 1, **caractérisé en ce qu'**il comprend mettre à disposition des mouvements de simulation de la mâchoire inférieure virtuelle à partir d'une position d'occlusion centrée ou habituelle, relation centrée, pour le planning virtuel d'opérations chirurgicales orthognatiques pour la correction de tailles d'os de mâchoire, restauration d'os, éléments d'articulations temporo-mandibulaires.

13. Procédé selon la revendication 1, comprenant en outre planning virtuel causal commun d'une correction orthognatique de la taille, forme et position d'os de mâchoire, d'éléments d'articulations temporo-mandibulaires, de correction orthodontique de position de dents, planning virtuel d'implantation en position d'occlusion centrée ou habituelle ou dans une position virtuellement planifiée de la relation centrée tenant compte des mouvements de simulation de la mâchoire inférieure virtuelle, et mettant à disposition la conception et par la suite la fabrication de dentures artificielles, applications et appareils orthodontiques, dispositifs pour la retenue de fragments d'os, des gabarits de guidage, des implants individuels pour la restauration d'os, des implants individuels pour le remplacement de dents manquants, des goupilles de repositionnement et de remède commandant la position de la mâchoire inférieure.

14. Procédé selon la revendication 1, comprenant en outre mettre à disposition des mouvements de simulation de la mâchoire inférieure virtuelle à partir d'une deuxième position fixée vers une position finale moyennant des calculs sur la base de données connues sur le déplacement d'une mâchoire inférieure à partir d'une première position fixée vers la deuxième position fixée et le déplacement de la première position fixée vers la position finale.

15. Moyen non volatile pouvant être lu par une machine et ayant un code enregistré contenant des informations pour la mise en œuvre du procédé selon la revendication 1.
